Europäisches Patentamt

**European Patent Office**

**Office européen des brevets**

(11) Numéro de publication: **0 277 868 B1**

# FASCICULE DE BREVET EUROPEEN

(45) Date de publication de fascicule du brevet: **20.05.92**

(51) Int. Cl.5: **A61M 1/16**, A61M 1/28, A61K 37/02, A61K 31/195

(21) Numéro de dépôt: **88400151.2**

(22) Date de dépôt: **25.01.88**

---

(54) **Solution pour dialyses et utilisation de peptides à base de glycine pour sa preparation.**

---

(30) Priorité: **27.01.87 GR 870129**

(43) Date de publication de la demande:
**10.08.88 Bulletin 88/32**

(45) Mention de la délivrance du brevet:
**20.05.92 Bulletin 92/21**

(84) Etats contractants désignés:
**AT BE CH DE ES FR GB IT LI LU NL SE**

(56) Documents cités:
**EP-A- 0 218 900**
**WO-A-87/01286**
**US-A- 4 339 433**

**ASAIO TRANSACTIONS. vol. 32, no. 1, 1986, HAGERSTOWN, MD US pages 550 - 553; E.Klein et al.: "Peptides as Substitute Osmotic Agents for Glucose in Peritoneal Dialysate"**

(73) Titulaire: **PIERRE FABRE MEDICAMENT**
**45, Place Abel Gance**
**F-92100 Boulogne(FR)**

(72) Inventeur: **Yatzidis, Hippocrates**
**Chatziyianni Mexi 3**
**Athenes(GR)**

(74) Mandataire: **Ahner, Francis et al**
**CABINET REGIMBEAU, 26, avenue Kléber**
**F-75116 Paris(FR)**

---

**Description**

La présente invention concerne une solution pour hémodialyse ou dialyse péritonéale.

La dialyse est utilisée comme méthode d'épuration extra-rénale pour combattre l'insuffisance rénale : le sang du malade, surchargé en catabolites (ou déchets) est mis en contact avec des solutions artificielles à travers une membrane.

De telles solutions artificielles doivent avoir une formule électrolytique se rapprochant le plus possible de celle du plasma normal et comportent, par exemple, des ions sodium, chlorure, potassium, calcium et/ou magnésium. A titre d'agent osmotique on utilise souvent le glucose. A titre d'agents tampons, on utilise les ions acétates, lactates et surtout les ions bicarbonates, ces derniers permettant de combattre l'acidose métabolique accompagnant fréquemment les insuffisances rénales.

Or, en présence d'acide lactique ou acétique et à un pH d'environ 5,5 les ions bicarbonates forment avec les ions calcium ou magnésium des complexes de la forme $Ca(HCO_3)_2$ ou $Mg(HCO_3)_2$.

Ces complexes sont solubles au départ, mais leur accumulation engendre une perte continue en dioxyde de carbone ($CO_2$) qui conduit à l'augmentation du pH.

Aussitôt que le pH est supérieur ou égal à environ 7,5, les complexes de calcium et de magnésium se transforment et précipitent sous la forme de carbonates ($Ca CO_3$ ou $Mg CO_3$) pratiquement insolubles.

Pour éviter ces précipitations, on a essayé de supprimer tous les ions bicarbonates et de ne conserver que les agents générant in vivo lesdits bicarbonates, tels que les ions acétates ou lactates.

La transformation de ces ions en bicarbonates n'est cependant pas totale ; de plus, il s'ensuit une accumulation d'acétates ou de lactates qui contribue à l'augmentation de l'acidose du malade, l'apparition de troubles du métabolisme et des troubles hémodynamiques.

D'autres procédés ont été envisagés pour éviter la formation de carbonates insolubles :

- le barbotage continu de $CO_2$ dans la solution de dialyse, pour maintenir le pH en dessous de 7,5 et éviter ainsi la formation et la précipitation de carbonates de calcium ou de magnésium ;
- la préparation de solutions pour dialyse exemptes d'ions calcium et magnésium et l'administration intra-veineuse séparée et simultanée de ces ions ;
- la préparation de solutés séparés, dont l'un, alcalin, contient les bicarbonates et l'autre, acide, comporte le calcium et le magnésium, ces deux solutés étant mélangés au moment de la dialyse ;
- l'addition de complexants du calcium et du magnésium, susceptibles de prévenir leur précipitation.

Tous ces procédés sont non seulement longs, compliqués et dangereux, mais nécessitent aussi l'utilisation d'appareillages spécifiques et coûteux ; en outre, aucun de ces procédés ne conduit de façon certaine à l'élimination des précipités de carbonates dans l'appareil du rein artificiel ou dans la cavité péritonéale ; enfin l'application de tels procédés est particulièrement problématique dans le cas de la dialyse péritonéale continue ambulatoire qui nécessite environ 4 échanges par 24 heures, des dialysats stériles et des conditions rigoureusement aseptiques.

Pour surmonter tous les inconvénients de la technique antérieure, la présente invention propose une nouvelle solution aqueuse stable d'électrolytes pour dialyses à base d'ions bicarbonates et comportant un peptide à base de glycine tels que de la glycylglycine, ainsi que les tri-, tétra-, penta-, et/ou hexaglycines. La solution pour dialyses de la présente invention comporte de préférence 5 à 100 mmoles/l de glycylglycine.

Un tel peptide confère à la solution pour dialyses une grande stabilité, grâce à un pH avoisinant le pH physiologique(7,35 ± 0,005)lui conférant un pouvoir tampon ; le pH de la solution pour dialyses selon l'invention est compris entre 6,75 et 8,00 et est, de préférence, compris entre 7,10 et 7,60.

Outre le peptide à base de glycine, la solution de la présente invention comporte tous les constituants habituellement utilisés pour la préparation d'une solution pour dialyses ; ces constituants peuvent notamment être choisis parmi les ions sodium, chlorure, potassium, calcium, magnésium, le glucose et/ou leurs mélanges ; mais d'autres constituants peuvent être utilisés tels que les ions sulfates ou citrates. Le pH et la stabilité de la solution de la présente invention dépendent, bien évidemment des quantités respectives de ses divers constituants et, en particulier, de la quantité de peptide à base de glycine qui est incorporée.

En outre, ce peptide s'est avéré jouer aussi le rôle d'agent osmotique et donc, sa quantité dans la solution de la présente invention est inversement proportionnelle à celle d'agent osmotique tel que le glucose.

C'est pourquoi, la présente invention concerne aussi les solutions pour dialyse ne comportant pas ou comportant peu de glucose.

Ce fait constitue an avantage majeur notamment dans le cas de la dialyse péritonéale continue où le glucose s'est révélé être un agent favorisant l'hyperglycémie du malade et donc l'artériosclérose.

La présente invention s'étend bien évidemment à l'utilisation de peptides à base de glycine, et notamment de glycylgcine pour la préparation de composition pour dialyse.

Selon la présente invention, il est aussi possible de préparer extemporanément une solution plus particulièrement destinée à l'hémodialyse et ce, à partir d'un mélange de poudres. C'est pourquoi la présente invention s'étend à l'utilisation d'un peptide à base de glycine sous forme de poudre pour la préparation extemporanée d'une telle solution. Les autres constituants de la solution sont dans ce cas introduits aussi sous fome de poudre.

Les tableaux suivants présentent des exemples de solutions selon la présente invention.

Ces solutions ont une formule électrolytique similare à celle des dialysats à base de lactate ou d'acétate, existant sur le marché.

Ces exemples ne limitent aucunement la portée de la présente invention, étant entendu que toutes les solutions pour dialyse à base de bicarbonates et comportant de la glycyclglycine et en particulier celles dont le pH est compris entre 6,75 et 7,60 font partie intégrante de cette description.

En particulier, des études minutieses entreprises dans le cadre de la présente invention ont motré que la substitutioln des chlorures de calcium et de magnésium par des citrates de calcium et de magnésium est avantageuse et cliniquement efficace pour l'hémodialyse. Une telle substitution confère aux solutions de la présente invention, une résistance accrue aux processus éventuels de cristallisation de carbonates, et par conséquent, une stabilité accrue.

En outre, il s'est avéré qu'une légère diminution de la concentration en bicarbonates (par exemple à 30 mmol/l) et/ou une éventuelle augmentation de la concentration en glycylglycine contribuent à améliorer la stabilité des solutions de la présente invention et sont notamment utiles pour prévenir une éventuelle alcalose en dialyse péritonéale continue ambulatoire.

Le tableau 1 présente trois solutions (a-, b-, et c-) pour dialyses comportant 10 mmoles/l de glycylglycine ($C_4H_8N_2O_3$) et dont le pH est compris entre 7,30 et 7,40. Les trois solutions se distinguent par leur teneur en glucose ($C_6H_{12}O_6$). On les désigne respectivement a-, b- et c-selon que leur teneur en glucose est de 15, 25 et 42,5 g/l.

On peut conserver de telles solutions pour dialyses en poches plastiques et dans des conditions stériles.

Ces solutions restent stables pendant plus de 18 mois.

Leur pH ne se modifie pas et leur teneur en ions calcium, magnésium et bicarbonates reste identique à lui-même pendant la durée de conservation. Ces solutions gardent leur limpidité ; aucun précipité de carbonates n'apparaît.

Le tableau 2 présente une solution pour dialyse dont le pH est compris entre 7,0 et 7,1 et qui comporte 50 mmol/l de glycylglycine. Cette solution sera désignée ci-après par la lettre d-. L'osmolalité de cette solution est d'environ 414,00.

La stabilité de cette solution est supérieure à 18 mois.

Des mesures in vitro prouvent qu'une telle solution pour dialyse augmente considérablement l'ultrafiltration, de sorte qu'une seule dialyse par 24 heures (au lieu de quatre) pourrait suffire en cas de dialyse péritonéale continue ambulatoire.

Le tableau 3 présente une solution selon l'invention comportant 5 mmoles/l de glycylglycine, dont le pH est compris entre 7,45 et 7,55 et qui sera désignée ci-après par la lettre e-.

Une telle formulation n'est véritablement stable que pendant un mois mais présente une efficacité biologique particulièrement performante dans le cas de troubles circulatoires. Une telle formulation est plus particulièrement destinée à l'hémodialyse.

EP 0 277 868 B1

TABLEAU 1

| Composants | g/l | $Na^+$ | $Cl^-$ | $K^+$ | $Ca^{2+}$ | $Mg^{2+}$ | $HCO_3^-$ | $C_4H_8N_2O_3$ | $C_6H_{12}O_6$ |
|---|---|---|---|---|---|---|---|---|---|
| | | | | | | | | mmol/l | |
| NaCl | 5,9034 | 101,00 | 101,00 | | | | | | |
| $NaHCO_3$ | 2,9403 | 35,00 | | | | | 35,00 | | |
| KCl | 0,0745 | | 1,00 | 1,00 | | | | | |
| $C_4H_8N_2O_3$ | 1,3212 | | | | | | | 10,00 | |
| $(CaCl_2,2H_2O)$ | 0,2572 | | 3,50 | | 1,75 | | | | |
| $(MgCl_2,6H_2O)$ | 0,1016 | | 1,00 | | | 0,50 | | | |
| $C_6H_{12}O_6$ | a- 15,0000 | | | | | | | | 83,25 |
| | b- 25,0000 | | | | | | | | 138,75 |
| | c- 42,5000 | | | | | | | | 236,00 |
| | | 136,00 | 106,50 | 1,00 | 1,75 | 0,50 | 35,00 | 10,00 | " |

pH : 7,35 ± 0,05

mmol/l :  
a- 374,00  
b- 429,50  
c- 526,75

EP 0 277 868 B1

TABLEAU 2

| Composants | g/l | mmol/l | | | | | | | |
|---|---|---|---|---|---|---|---|---|---|
| | | $Na^+$ | $Cl^-$ | $K^+$ | $Ca^{2+}$ | $Mg^{2+}$ | $HCO_3^-$ | $C_4H_8N_2O_3$ | $C_6H_{12}O_6$ |
| NaCl | 5,9034 | 101,00 | 101,00 | | | | | | |
| $NaHCO_3$ | 2,9403 | 35,00 | | | | | 35,00 | | |
| KCl | 0,0745 | | 1,00 | 1,00 | | | | | |
| $C_4H_8N_2O_3$ | 6,6060 | | | | | | | 50,00 | |
| $(CaCl_2, 2H_2O)$ | 0,2572 | | 3,50 | | 1,75 | | | | |
| $(MgCl_2, 6H_2O)$ | 0,1016 | | 1,00 | | | 0,50 | | | |
| $C_6H_{12}O_6$ | 15,0000 | | | | | | | | 83,25 |
| | | 136,00 | 106,50 | 1,00 | 1,75 | 0,50 | 35,00 | 50,00 | 83,25 |

PH : 7,05 $\pm$ 0,05        mmol/l : d- 4 14,00

EP 0 277 868 B1

## TABLEAU 3

| Substances | g/l | mmol/l | | | | | | |
|---|---|---|---|---|---|---|---|---|
| | | $Na^+$ | $Cl^-$ | $K^+$ | $Ca^{2+}$ | $Mg^{2+}$ | $HCO_3^-$ | $C_4H_8N_2O_3$ |
| NaCl | 6,0495 | 103,50 | 103,50 | | | | | |
| $NaHCO_3$ | 2,9403 | 35,00 | | | | | 35,00 | |
| KCl | 0,1118 | | 1,50 | 1,50 | | | | |
| $C_4H_8N_2O_3$ | 0,6606 | | | | | | | 5,00 |
| $(CaCl_2,2H_2O)$ | 0,2572 | | 3,50 | | 1,75 | | | |
| $(MgCl_2,6H_2O)$ | 0,1016 | | 1,00 | | | 0,50 | | |
| | | 138,50 | 109,50 | 1,50 | 1,75 | 0,50 | 35,00 | 5,00 |

pH : 7,50 ± 0,05          mmol/l : e- 291,75

Les exemples suivants illustrent la présente invention sans aucunement en limiter la portée.

EXEMPLE 1 :

Préparation des dialysats

Chaque solution pour dialyse a été préparée par dilution de substances chimiques de bonne qualité dans de l'eau très pure produite par la méthode d'osmose inverse combinée et déminéralisation (Millipore/Milli-Q). (La Société Merck a fourni le chlorure de sodium, le bicarbonate de sodium, le chlorure de potassium, le chlorure de calcium dihydraté, le chlorure de magnésium hexahydraté. Le glucose anhydre provient de MALLINCK RODT. La glycylglycine ou diglycine provient de SERVA et la Société SIGMA a fourni la glycylglycylglycine ou triglycine).

Puis les dialysats a-, b-, c- et d- sont filtrés au moyen d'ultrafiltres stériles (PORTEX) et transférés dans des flacons ajustables rapidement, équipés de bouchons en caoutchouc étanches à l'air. Les flacons et bouchons sont stérilisés à 115°C pendant 3 heures. Ils sont stockés à une température ambiante comprise entre 10 et 40°C.

Le dialysat e- est stocké sans aucune précaution particulière à la température de la pièce et exposé à l'air.

EXEMPLE 2

Stabilité des dialysats

2.1. Méthodes

Dans le but d'évaluer la stabilité des dialysats, on prélève, par aspiration, l'échantillon de manière aseptique et anaérobique ( on place avec précaution la seringue au niveau de la surface de la solution se trouvant dans les flacons). Ce prélèvement est effectué à divers intervalles de temps et immédiatement après la préparation décrit à l'exemple 1.

Des études biochimiques sont effectuées immédiatement. On mesure le pH, la quantité d'HCO$_3$ - (Radiomètre, ABL-2), l'osmolalité (par détermination du point de congélation - Gonotec-Osmomat 030), la quantité de calcium et de magnésium par spectrophotométrie d'absorption (Perkin-Elmer 1370). La glycine et la glycylglycine sont dosées par une méthode modifiée à la ninhydrine, réalisée dans des échantillons respectivement hydrolysés (6 moles D'HCl, 24 heures à 110°C) et non hydrolysés de dialysat.

La méthode est spécifique de la glycine libre, ne donnant aucune couleur pour les peptides à base de glycine.

2.2. Résultats

Les résultats remarquables obtenus sont exposés dans les tableaux suivants.

Les tableaux 4, 5 et 6 correspondent respectivement aux dialysats a-, b- et c-.

Le tableau 7 donne les résultats de stabilité du dialysat d-.

Le tableau 8 donne les résultats de stabilité du dialysat e-.

## Tableau 4

| Mois | mOsmol/kg Osmolalité | pH | mmol/l HCO$_3^-$ | Ca$^+$ | Mg$^+$ | Glycyl-glycine | Gly-cine |
|------|------|------|------|------|------|------|------|
| 0 | 372,0 | 7,30 | 32,8 | 1,76 | 0,50 | 10,00 | nul |
| 1 | 370 0 | 7,32 | 33,0 | 1,77 | 0,51 | 9,89 | " |
| 2 | 374,0 | 7,29 | 33,0 | 1,74 | 0,52 | 10,05 | " |
| 3 | 376,5 | 7,31 | 32,4 | 1,72 | 0,50 | 9,95 | " |
| 4 | 375,0 | 7,28 | 33,2 | 1,76 | 0,48 | 10,00 | " |
| 5 | 371,5 | 7,30 | 32,5 | 1,72 | 0,54 | 10,05 | " |
| 6 | 376,0 | 7,34 | 32,0 | 1,77 | 0,50 | 10,00 | " |
| 7 | 376,6 | 7,30 | 31,8 | 1,69 | 0,50 | 9,92 | " |
| 8 | 370,0 | 7,28 | 33,0 | 1,75 | 0,51 | 10,00 | " |
| 9 | 374,0 | 7,31 | 32,2 | 1,75 | 0,49 | 9,88 | " |
| 10 | 370,0 | 7,33 | 32,4 | 1,72 | 0,48 | 10,02 | " |
| 11 | 375,0 | 7,30 | 33,0 | 1,76 | 0,52 | 9,90 | " |
| 12 | 371,5 | 7,31 | 32,0 | 1,72 | 0,50 | 9,95 | " |
| 13 | 373,0 | 7,33 | 33,5 | 1,76 | 0,50 | 9,90 | " |
| 14 | 372,0 | 7,30 | 31,5 | 1,72 | 0,53 | 10,00 | " |
| 15 | 370,0 | 7,28 | 32,5 | 1,75 | 0,51 | 10,00 | " |
| 16 | 376,0 | 7,31 | 34,0 | 1,77 | 0,50 | 10,00 | " |

Tableau 5

| Mois | mOsmol/kg Osmolalité | pH | mmol/l HCO$_3^-$ | Ca$^+$ | Mg$^+$ | Glycyl- glycine | Gly- cine |
|---|---|---|---|---|---|---|---|
| 0 | 426,0 | 7,32 | 33,5 | 1,74 | 0,51 | 10,00 | Nul |
| 1 | 430,0 | 7,30 | 32,5 | 1,76 | 0,50 | 9,92 | " |
| 2 | 425,0 | 7,27 | 32,0 | 1,72 | 0,50 | 10,00 | " |
| 3 | 426,0 | 7,33 | 31,8 | 1,77 | 0,52 | 9,95 | " |
| 4 | 430,0 | 7,31 | 32,2 | 1,75 | 0,49 | 10,05 | " |
| 5 | 418,0 | 7,30 | 33,0 | 1,73 | 0,50 | 10,02 | " |
| 6 | 425,0 | 7,29 | 32,0 | 1,76 | 0,50 | 9,96 | " |
| 7 | 424,0 | 7,28 | 32,5 | 1,71 | 0,51 | 10,00 | " |
| 8 | 425,0 | 7,32 | 32,0 | 1,76 | 0,49 | 10,00 | " |
| 9 | 430,0 | 7,32 | 31,8 | 1,72 | 0,51 | 10,00 | " |
| 10 | 423,0 | 7,30 | 32,4 | 1,72 | 0,48 | 9,95 | " |
| 11 | 430,0 | 7,32 | 33,0 | 1,74 | 0,52 | 10,05 | " |
| 12 | 425,0 | 7,30 | 32,0 | 1,76 | 0,50 | 10,00 | " |
| 13 | 428,0 | 7,29 | 33,2 | 1,77 | 0,51 | 9,88 | " |
| 14 | 430,0 | 7,32 | 33,0 | 1,74 | 0,49 | 9,98 | " |
| 15 | 416,0 | 7,30 | 32,0 | 1,73 | 0,51 | 10,05 | " |

## Tableau 6

| Mois | mOsmol/kg Osmolalité | pH | mmol/1 HCO₃⁻ | Ca+ | Mg+ | Glycyl-glycine | Gly-cine |
|---|---|---|---|---|---|---|---|
| 0 | 525,0 | 7,31 | 33,0 | 1,77 | 0,50 | 9,95 | nul |
| 1 | 528,0 | 7,30 | 32,8 | 1,75 | 0,50 | 10,05 | " |
| 2 | 531,0 | 7,28 | 32,5 | 1,72 | 0,50 | 10,00 | " |
| 3 | 520,0 | 7,28 | 32,0 | 1,77 | 0,48 | 10,00 | " |
| 4 | 423,0 | 7,28 | 32,5 | 1,75 | 0,51 | 9,98 | " |
| 5 | 531,0 | 7,29 | 32,3 | 1,76 | 0,52 | 9,95 | " |
| 6 | 514,0 | 7,30 | 31,8 | 1,74 | 0,50 | 9,95 | " |
| 7 | 517,0 | 7,31 | 32,0 | 1,75 | 0,50 | 10,05 | " |
| 8 | 530,0 | 7,30 | 32,5 | 1,72 | 0,48 | 10,00 | " |
| 9 | 523,0 | 7,28 | 32,0 | 1,74 | 0,49 | 10,00 | " |
| 10 | 521,0 | 7,30 | 32,0 | 1,72 | 0,50 | 10,05 | " |
| 11 | 532,0 | 7,31 | 32,6 | 1,76 | 0,52 | 9,90 | " |
| 12 | 531,0 | 7,29 | 32,0 | 1,74 | 0,50 | 9,92 | " |
| 13 | 516,0 | 7,31 | 31,8 | 1,75 | 0,50 | 9,98 | " |

Tableau 7

| Mois | mOsmol/kg Osmolalité | pH | mmol/l HCO$_3^-$ | Ca+ | Mg+ | Glycyl- glycine | Gly- cine |
|---|---|---|---|---|---|---|---|
| 0 | 415,5 | 7,10 | 32,8 | 1,74 | 0,50 | 50,5 | nul |
| 1 | 412,0 | 7,04 | 32,5 | 1,75 | 0,51 | 50,2 | " |
| 2 | 410,0 | 7,08 | 33,0 | 1,77 | 0,50 | 51,0 | " |
| 3 | 412,0 | 7,05 | 32,0 | 1,74 | 0,48 | 49,0 | " |
| 4 | 410,0 | 7,07 | 33,0 | 1,73 | 0,49 | 50,5 | " |
| 5 | 420,0 | 7,10 | 32,8 | 1,72 | 0,48 | 50,0 | " |
| 6 | 413,0 | 7,05 | 33,0 | 1,74 | 0,50 | 50,0 | " |
| 7 | 417,0 | 7,02 | 31,8 | 1,77 | 0,52 | 48,9 | " |
| 8 | 420,0 | 7,00 | 32,0 | 1,73 | 0,50 | 50,3 | " |
| 9 | 414,0 | 7,04 | 31,8 | 1,77 | 0,48 | 51,0 | " |
| 10 | 412,0 | 7,08 | 32,4 | 1,72 | 0,50 | 50,6 | " |
| 11 | 416,0 | 7,10 | 32,8 | 1,75 | 0,51 | 49,8 | " |
| 12 | 411,0 | 7,05 | 33,0 | 1,77 | 0,51 | 49,0 | " |
| 13 | 416,0 | 7,10 | 32,8 | 1,73 | 0,50 | 50,2 | " |
| 14 | 412,0 | 7,05 | 33,0 | 1,72 | 0,49 | 49,0 | " |
| 15 | 417,0 | 7,05 | 33,2 | 1,76 | 0,52 | 51,0 | " |
| 16 | 412,0 | 7,04 | 32,5 | 1,78 | 0,50 | 51,2 | " |
| 17 | 412,0 | 7,10 | 32,0 | 1,80 | 0,50 | 49,6 | " |
| 18 | 417,0 | 7,06 | 32,8 | 1,76 | 0,51 | 49,5 | " |

Tableau 8

| | mOsmol/kg | | | mmol/l | | | Glycyl- | Gly- |
|---|---|---|---|---|---|---|---|---|
| Jours | Osmolalité | pH | $HCO_3^-$ | $Ca^+$ | $Mg^+$ | | glycine | cine |
| 0 | 290,0 | 7,48 | 33,0 | 1,75 | 0,50 | | 5,00 | nul |
| 1 | 288,0 | 7,43 | 32,8 | 1,72 | 0,49 | | 4,96 | " |
| 2 | 282,0 | 7,48 | 33,5 | 1,75 | 0,50 | | 5,05 | " |
| 3 | 287,0 | 7,58 | 32,2 | 1,70 | 0,45 | | 4,98 | " |
| 4 | 286,0 | 7,65 | 31,8 | 1,68 | 0,42 | | 5,03 | " |
| 5 | 285,0 | 7,70 | 30,0 | 1,50 | 0,40 | | 5,00 | " |

Il s'est donc avéré que la glycylglycine permet de réaliser de véritables solutions tampons et d'assurer leur stabilité.

En effet, aucun changement appréciable du pH, des quantités en $HCO_3$, Ca, Mg et de l'osmolalité, n'a pu être détecté. La quantité de glycylglycine demeure inchangée.

La glycine n'apparait pas dans les échantillons non hydrolysés des dialysats a-, b-, c- et d- pendant au moins un an.

Les peptides à base de glycine sont très résistants aux processus de dégradation. Leur température de décomposition est supérieure à 270°C.

Le dialysat e- est moins stable du fait que son pH, environ égal à 7,50, est proche de la valeur critique (comprise entre 7,55 et 7,60) qui favorise la formation de carbonates de calcium et de magnésium insolubles.

EXEMPLE 3 :

Dialysat péritonéale chez l'animal

3.1. Méthodes

Dans le but d'évaluer la capacité d'ultra-filtration du dialysat comportant du bicarbonate et des peptides à base de glycine, on effectue une perfusion directe dans la cavité péritonéale de lapins, de 40 ml/kg du dialysat d- (1,5 % de glucose, 415 mosmol/kg).

On injecte aussi cette même quantité de dialysat dans lequel on a remplacé la diglycine (ou glycylglycine) par une quantité équimolaire de triglycine pour réaliser un essai supplémentaire (1,5 % de glucose, 415 mosmol/kg).

Le dialysat témoin utilisé est la solution Dianeal de la Société Travenol (2,27 % de glucose, 400 mosmol/kg).

La perfusion est réalisée chez 3 groupes séparés comportant chacun 4 lapins (d'environ 3 à 4 kg).

Après un temps de latence de 4 heures, les la~pins sont sacrifiés et le liquide péritonéal est collecté avec précaution. La membrane péritonéale est étudiée sur les plans microscopique et histologique.

3.2. Résultats

Tous les animaux ont bien toléré la perfusion péritonéale du dialysat à base de bicarbonate et de di- ou de triglycine. Il ressort des études microscopiques et histologique, qu'aucune inflammation du péritoine n'est apparue.

Le dialysat d- à base de bicarbonate et de glycylglycine a une osmolalité approximativement similaire à celle de la solution Dianeal de Travenol. Et, malgré le remplacement de molécules assez grosses de glucose de la solution Dianeal (180 daltons) par des molécules plus petites de diglycine du dialysat d- (132,12 daltons), le dialysat d-de la présente invention a permis une ultrafiltration presque égale à celle obtenue par la solution Dianeal, soit 7,40 versus 7,80 ml/kg pour le dialysat de l'invention versus la solution Dianeal.

En outre, le dialysat à base de bicarbonate et de triglycine produit significativement plus d'ultrafiltrat que la solution Dianeal, bien que les deux dialysats soient approximativement iso-osmotiques et que les tailles des molécules de glucose et de triglycine soient similaires (180 et 189,2 daltons respectivement).

Les quantités d'ultra-filtrat sont, en effet de 9,50 ml/kg versus 7,40 ml/kg pour les dialysats de l'invention et la solution Dianeal respectivement.

EXEMPLE 4

Hémodialyse chez l'homme

4.1. Méthodes

On évalue le cas de 4 patients, 2 hommes et 2 femmes âgés d'environ 40 à 65 ans, traités par hémodialyse avec une solution d'acétate standard (solution y-) pendant 2 à 4 ans et présentant, les 4 derniers mois, des effets secondaires importants : maux de tête, nausées, vomissements et hypotension.

On modifie le traitement de ces patients en utilisant le dialysat e- de la présente invention dans des conditions similaires à celles du traitement précédent : 3 fois par semaine pendant 4 heures (débit sanguin et du dialysat : 200 ml et 500 ml par minute, unité de cuprophane 1,0 m$^2$).

L'hémodialyse est réalisée à l'aide de l'appareil RSP/Travenol.

La composition des dialysats e- et y- est approximativement similaire quant aux concentrations en calcium, sodium, potassium et magnésium. Les différences entre ces deux dialysats consistent notamment en ce que les 35 mmoles/l d'acétate (du dialysat y-) ont été remplacés par 35 mmoles/l de bicarbonates et 5 mmoles/l de diglycine ( pour le dialysat e-).

Le contrôle biochimique est effectué une fois par semaine : au début de la dialyse, 1 heure après l'initiation, et immédiatement après la dialyse et ce, pendant 4 mois pour des patients traités respectivement par les dialysats y- et e-.

4.2. Résultats

Les patients hémodialysés ont bien toléré le dialysat e-à base de bicarbonate et de glycylglycine. Aucun effet secondaire n'a été noté : les incidents de maux de tête, vomissements, nausée et hypotension ont diminué progressivement puis complètement disparu à la fin du premier mois.

Le traitement au moyen du dialysat e- a donné une meilleure correction de l'acidose métabolique que le traitement au moyen du dialysat y-. Les résultats sont reproduits dans le tableau 9 suivant :

### Tableau 9

| | Acétate (dialysat y-) | | |
|---|---|---|---|
| | Début de la dialyse | 1 heure | Après la dialyse |
| pH | 7,340±0,045 | 7,350±0,037 | 7,380±0,058 |
| $HCO_3$ | 17,9±2,1 | 17,0±1,8 | 18,0±2,6 |
| | Bicarbonate-glycylglycine (dialysat e-) | | |
| | Début de la dialyse | 1 heure | Après la dialyse |
| pH | 7,385±0,020 | 7,420±0,030 | 7,465±0,030 |
| $HCO_3$ | 20,5±2,0 | 24,9±1,2 | 27,3±1,6 |
| $p <$ | 0,04 | 0,001 | 0,001 |

Une augmentation significative des valeurs de pH et $HCO_3$ a été obtenue pendant la dialyse à l'aide du dialysat e-, alors que durant la dialyse à l'aide du dialysat standard y-à base d'acétate, le pH a légèrement augmenté et la quantité d'$HCO_3$ est restée pratiquement inchangée.

Les valeurs en $pCO_2$ sont restées stables lors de la dialyse à l'aide de la solution e- alors qu'elles ont décru lors de la dialyse à l'acétate, principalement 1 heure après l'initiation et l'après-dialyse.

On n'a noté aucune différence relative aux concentrations sanguines en $pO_2$, Na, Cl, K, P, Ca, Mg, urée et créatine entre la dialyse avec la solution e- et la dialyse à l'acétate (y-).

Les quantités de perfusion à la glycylglycine sont, pour l'hémodialyse comprises entre 24 et 32 g par dialyse (versus 100 l de dialysat) contenant 5 mmoles/l et, pour l'hémodialyse péritonéale continue ambulatoire, inférieures à 7,5 g/24 heures (en utilisant 8 échanges 1/4 de 2 litres par jour avec une concentration de 10 mmoles/litre).

De telles quantités perfusées sont non seulement sans danger, mais peuvent être aussi bénéfiques.

Les quatre patients traités depuis plus de quatre mois par le dialysat e- sont en excellent état.

**Revendications**

**Revendications pour les Etats contractants suivants : AT, BE, CH, DE, FR, GB, IT, LI, LU, NL, SE**

**1.** Solution aqueuse stable d'électrolytes pour dialyses à base d'ions bicarbonates caractérisée en ce qu'elle comporte un peptide à base de glycine.

**2.** Solution selon la revendication 1, caractérisée en ce que le peptide à base de glycine est choisi parmi les di-, tri-, tétra-, penta- et/ou hexa- glycine.

**3.** Solution selon la revendication 2, caractérisée en ce que le peptide à base de glycine est la glycylglycine.

**4.** Solution selon la revendication 3, caractérisée en ce qu'elle comporte 5 à 100 mmoles/l de glycylglycine.

**5.** Solution selon l'une quelconque des revendications 1 à 4, caractérisée en ce que son pH est compris entre 6,75 et 8,00.

**6.** Solution selon la revendication 5, caractérisée en ce que son pH est compris entre 7,10 et 7,60.

**7.** Solution selon l'une des revendications 1 à 6, caractérisée en ce qu'elle comporte des ions choisis parmi les ions sodium, potassium, calcium, chlorure, magnésium et/ou leurs mélanges.

**8.** Solution selon l'une des revendications 1 à 7, caractérisée en ce qu'elle comporte du glucose.

**9.** Utilisation d'un peptide à base de glycine pour la préparation d'une solution pour dialyses.

**10.** Utilisation selon la revendication 9, caractérisée en ce que le peptide est la glycylglycine.

**11.** Utilisation d'un peptide à base de glycine sous forme de poudre pour la préparation extemporanée d'une solution selon l'une des revendications 1 à 8 plus particuliérement destinée à l'hémodialyse.

**12.** Utilisation selon la revendication 11, caractérisée en ce que les ions constitutifs de la solution pour hémodialyse sont aussi introduits sous forme de poudre lors de la préparation extemporanée.

**Revendications pour l'Etat contractant suivant : ES**

**1.** Procédé de préparation d'une solution aqueuse stable d'électrolytes pour dialyses, caractérisé en ce qu'on mélange des constituants comprenant des ions bicarbonates avec un peptide à base de glycine.

**2.** Procédé de préparation d'une solution selon la revendication 1, caractérisé en ce que le peptide à base de glycine est choisi parmi les di-, tri-, tétra-, penta- et/ou hexa- glycine.

**3.** Procédé de préparation d'une solution selon la revendication 2, caractérise en ce que le peptide à base de glycine est la glycylglycine.

**4.** Procédé de préparation d'une solution selon la revendication 3, caractérise en ce qu' elle comporte 5 à 100 mmoles/l de glycylglycine.

**5.** Procédé de préparation d'une solution selon l'une quelconque des revendications 1 à 4, caractérisé en ce que le pH de la solution est compris entre 6,75 et 8,00.

**6.** Procédé de préparation d'une solution selon la revendication 5, caractérisé en ce que le pH de la solution est compris entre 7,10 et 7,60.

**7.** Procédé de préparation d'une solution selon l'une des revendications 1 à 6, caractérisé en ce qu'elle comporte des ions choisis parmi les ions sodium, potassium, calcium, chlorure, magnésium et/ou leurs mélanges.

**8.** Procédé de préparation d'une solution selon l'une quelconque des revendications 1 à 7, caractérisé en ce qu'elle comporte du glucose.

**9.** Utilisation d'un peptide à base de glycine pour la préparation d'une solution pour dialyses.

15

**10.** Utilisation selon la revendication 9, caractérisée en ce que le peptide est la glycylglycine.

**11.** Utilisation d'un peptide à base de glycine sous forme de poudre pour la préparation extemporanée d'une solution selon l'une des revendications 1 à 8 plus particulièrement destinée à l'hémodialyse.

**12.** Utilisation selon la revendication 11, caractérisée en ce que les ions constitutifs de la solution pour hémodialyse sont aussi introduits sous forme de poudre lors de la préparation extemporanée.

**Claims**

**Claims for the following Contracting States : AT, BE, CH, DE, FR, GB, IT, LI, LU, NL, SE**

**1.** A stable aqueous solution of electrolytes for dialysis, based on bicarbonate ions, characterized in that it contains a peptide based on glycine.

**2.** Solution according to claim 1, characterized in that the peptide based on glycine is chosen from di-, tri-, tetra-, penta- and/or hexaglycine.

**3.** Solution according to claim 2, characterized in that the peptide based on glycine is glycylglycine.

**4.** Solution according to claim 3, characterized in that it contains 5 to 100 mmol/l of glycylglycine.

**5.** Solution according to any one of the claims 1 to 4, characterized in that its pH is between 6.75 and 8.00.

**6.** Solution according to claim 5, characterized in that its pH is between 7.10 and 7.60.

**7.** Solution according to one of the claims 1 to 6, characterized in that it contains ions chosen from sodium, potassium, calcium, chloride and magnesium ions and/or the mixtures thereof.

**8.** Solution according to any one of the claims 1 to 7, characterized in that it contains glucose.

**9.** The use of a peptide based on glycine for the preparation of a solution for dialysis.

**10.** Use according to claim 9, characterized in that the peptide is glycylglycine.

**11.** The use of a peptide based on glycine in powder form for the preparation at the time of use of a solution, according to one of the claims 1 to 8, intended more especially for hemodialysis.

**12.** Use according to claim 11, characterized in that the constituent ions of the solution for hemodialysis are also introduced in powder form during the preparation at the time of use.

**Claims for the following Contracting State : ES**

**1.** Process for preparing a stable aqueous solution of electrolytes for dialysis, characterized in that constituents, comprising bicarbonate ions, with a peptide based on glycine, are mixed.

**2.** Process for preparing a solution according to claim 1, characterized in that the peptide based on glycine is chosen from di-, tri-, tetra-, penta- and/or hexaglycine.

**3.** Process for preparing a solution according to claim 2, characterized in that the peptide based on glycine is glycylglycine.

**4.** Process for preparing a solution according to claim 3, characterized in that it contains 5 to 100 mmol/l of glycylglycine.

**5.** Process for preparing a solution according to any one of the claims 1 to 4, characterized in that the pH of the solution is between 6.75 and 8.00.

6. Process for preparing a solution according to claim 5, characterized in that the pH of the solution is between 7.10 and 7.60.

7. Process for preparing a solution according to one of the claims 1 to 6, characterized in that it contains ions chosen from sodium, potassium, calcium, chloride and magnesium ions and/or the mixtures thereof.

8. Process for preparing a solution according to any one of the claims 1 to 7, characterized in that it contains glucose.

9. The use of a peptide based on glycine for the preparation of a solution for dialysis.

10. Use according to claim 9, characterized in that the peptide is glycylglycine.

11. The use of a peptide based on glycine in powder form for the preparation at the time of use of a solution, according to one of the claims 1 to 8, intended more especially for hemodialysis.

12. Use according to claim 11, characterized in that the constituent ions of the solution for hemodialysis are also introduced in powder form during the preparation at the time of use.

**Patentansprüche**

**Patentansprüche für folgende Vertragsstaaten : AT, BE, CH, DE, FR, GB, IT, LI, LU, NL, SE**

1. Stabile wäßrige Lösung von Elektrolyten für Dialysen, auf der Basis von Bicarbonationen, dadurch gekennzeichnet, daß sie ein Peptid auf der Basis von Glycin enthält.

2. Lösung nach Anspruch 1, dadurch gekennzeichnet, daß das Peptid auf der Basis von Glycin ausgewählt ist aus Di-, Tri-, Tetra-, Penta- und/oder Hexa-glycin.

3. Lösung nach Anspruch 2, dadurch gekennzeichnet, daß das Peptid auf der Basis von Glycin Glycylglycin ist.

4. Lösung nach Anspruch 3, dadurch gekennzeichnet, daß sie 5 bis 100 mmol/l Glycylglycin enthält.

5. Lösung nach einem der Ansprüche 1 bis 4, dadurch gekennzeichnet, daß ihr pH bei 6,75 bis 8,00 liegt.

6. Lösung nach Anspruch 5, dadurch gekennzeichnet, daß ihr pH bei 7,10 bis 7,60 liegt.

7. Lösung nach einem der Ansprüche 1 bis 6, dadurch gekennzeichnet, daß sie Ionen umfaßt, die ausgewählt sind aus den Ionen von Natrium, Kalium, Calcium, Chlorid, Magnesium und/oder ihren Gemischen.

8. Lösung nach einem der Ansprüche 1 bis 7, dadurch gekennzeichnet, daß sie Glucose umfaßt.

9. Verwendung eines Peptids auf der Basis von Glycin zur Herstellung einer Lösung für Dialysen.

10. Verwendung nach Anspruch 9, dadurch gekennzeichnet, daß das Peptid Glycylglycin ist.

11. Verwendung eines Peptids auf der Basis von Glycin in Pulverform zur unmittelbaren Herstellung einer Lösung nach einem der Ansprüche 1 bis 8, insbesondere bestimmt zur Hämodialyse.

12. Verwendung nach Anspruch 11, dadurch gekennzeichnet, daß die die Lösung für die Hämodialyse bildenden Ionen auch in Pulverform bei der unmittelbaren Herstellung eingebracht werden.

**Patentansprüche für folgenden Vertragsstaat : ES**

1.  Verfahren zur Herstellung einer stabilen, wäßrigen Lösung von Elektrolyten für Dialysen, dadurch gekennzeichnet, daß man Bestandteile, die Bicarbonationen umfassen, mit einem Peptid auf der Basis von Glycin vermischt.

2.  Verfahren zur Herstellung einer Lösung nach Anspruch 1, dadurch gekennzeichnet, daß das Peptid auf der Basis von Glycin ausgewählt wird aus Di-, Tri-, Tetra-, Penta- und/oder Hexa-glycin.

3.  Verfahren zur Herstellung einer Lösung nach Anspruch 2, dadurch gekennzeichnet, daß das Peptid auf der Basis von Glycin Glycylglycin ist.

4.  Verfahren zur Herstellung einer Lösung nach Anspruch 3, dadurch gekennzeichnet, daß sie 5 bis 100 mmol/l Glycylglycin umfaßt.

5.  Verfahren zur Herstellung einer Lösung nach einem der Ansprüche 1 bis 4, dadurch gekennzeichnet, daß der pH der Lösung bei 6,75 bis 8,00 liegt.

6.  Verfahren zur Herstellung einer Lösung nach Anspruch 5, dadurch gekennzeichnet, daß der pH der Lösung bei 7,10 bis 7,60 liegt.

7.  Verfahren zur Herstellung einer Lösung nach einem der Ansprüche 1 bis 6, dadurch gekennzeichnet, daß sie Ionen umfaßt, die ausgewählt werden aus den Ionen von Natrium, Kalium, Calcium, Chlorid, Magnesium und/oder deren Gemischen.

8.  Verfahren zur Herstellung einer Lösung nach einem der Ansprüche 1 bis 7, dadurch gekennzeichnet, daß sie Glucose umfaßt.

9.  Verwendung eines Peptids auf der Basis von Glycin zur Herstellung einer Lösung für Dialysen.

10. Verwendung nach Anspruch 9, dadurch gekennzeichnet, daß das Peptid Glycylglycin ist.

11. Verwendung eines Peptids auf der Basis von Glycin in Pulverform zur unmittelbaren Herstellung einer Lösung nach einem der Ansprüche 1 bis 8, insbesondere bestimmt zur Hämodialyse.

12. Verwendung nach Anspruch 11, dadurch gekennzeichnet, daß die die Lösung zur Hämodialyse bildenden Ionen auch in Pulverform bei der unmittelbaren Herstellung eingebracht werden.